(19) Europäisches Patentamt
European Patent Office
Office européen des brevets

(11) Publication number: **0 524 525 A1**

(12) **EUROPEAN PATENT APPLICATION**

(21) Application number: **92111990.5**

(22) Date of filing: **14.07.92**

(51) Int. Cl.[5]: **C07C 323/47**, C07C 317/28, A01N 41/10, A01N 35/10

(30) Priority: **26.07.91 JP 208807/91**

(43) Date of publication of application: **27.01.93 Bulletin 93/04**

(84) Designated Contracting States: **BE CH DE FR GB IT LI NL**

(71) Applicant: **NIHON BAYER AGROCHEM K.K.**
**7-1, Nihonbashi Honcho 2-chome Chuo-ku**
**Tokyo 103(JP)**

(72) Inventor: **Goto, Toshio**
**214-18, Koganei, Kokubunji-machi**
**Shimotsuga-gun, Tochigi(JP)**
Inventor: **Yanagi, Akihiko**
**1-21-1, Ekiminami-machi**
**Oyama-shi, Tochigi(JP)**
Inventor: **Hayakawa, Hidenori**
**3-17-20, Ekiminami-machi**
**Oyama-shi, Tochigi(JP)**
Inventor: **Asami, Tadao**
**1-37, Nijyukki-machi**
**Shinjyuku-ku, Tokyo(JP)**

(74) Representative: **Schumacher, Günter, Dr. et al**
**c/o Bayer AG Konzernverwaltung RP**
**Patentabteilung**
**W-5090 Leverkusen 1 Bayerwerk(DE)**

(54) **Herbicidally active 3-hydroxy-2-cyclohexen-1-ones.**

(57) Novel 3-hydroxy-2-cyclohexen-1-ones of the formula (I)

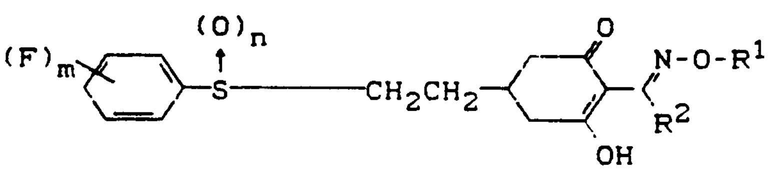

(I)

wherein

- $R^1$ represents $C_{1-4}$-alkyl group, cycloalkylalkyl with up to 6 carbon atoms in this cycloalkyl moeity and up to 4 carbon atoms in the alkyl moeity, $C_{3-4}$ alkenyl which may be substituted by halogen or $C_{3-4}$ alkynyl group,
- $R^2$ represents $C_{1-4}$-alkyl group,
- m represents 2 or 3, and
- n represents 0 or 2,

and the use of the new compounds as herbicides.

The present invention relates to novel 3-hydroxy-2-cyclohexen-1-ones, to processes for their preparation and to their use as herbicides.

It has already been disclosed that a certain group of cyclohexenes has herbicidal activities (see Japanese Patent Laid-open Nos. 115349/1979 and 146856/1988).

There have now been found novel 3-hydroxy-2-cyclohexen-1-ones of the formula (I)

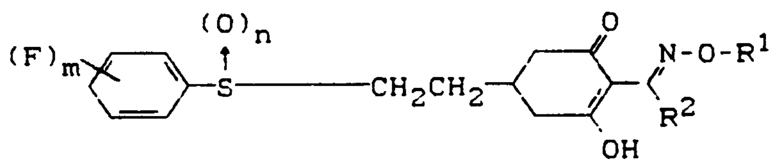

(I)

wherein

- $R^1$ represents $C_{1-4}$-alkyl group, cycloalkylalkyl with up to 6 carbon atoms in the cycloalkyl moeity and up to 4 carbon atoms in the alkyl moeity, $C_{3-4}$ alkenyl which may be substituted by halogen or $C_{3-4}$ alkynyl group,
- $R^2$ represents $C_{1-4}$-alkyl group,
- m represents 2 or 3, and
- n represents 0 or 2.

The compounds of the formula (I) can be obtained by a process in which

a) compounds of the formula (II)

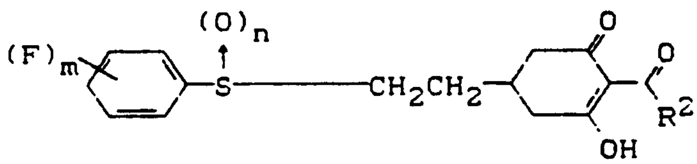

(II)

wherein

$R^2$, m and n have the same meanings as mentioned above, are reacted with compounds of the formula (III)

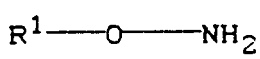

(III)

wherein

$R^1$ has the same meaning as mentioned above, in the presence of inert solvents,

or

b) where n is 2: compounds of the formula (Ia)

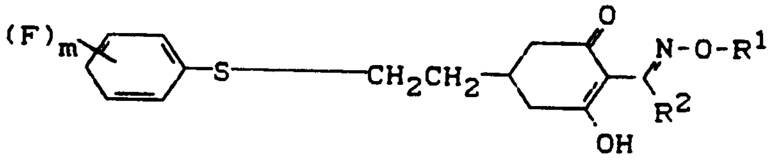

(Ia)

wherein $R^1$, $R^2$ and m have the same meanings as mentioned above,
are oxidized in the presence of inert solvents.

The 3-hydroxy-2-cyclohexen-1-ones exhibit powerful herbicidal properties.

The 3-hydroxy-2-cyclohexen-1-ones according to the present invention are included in the conception of the invention according to Japanese Patent Laid-open No. 115349/1979 but the compounds according to

the present invention are novel compounds that have not specifically been disclosed.

Surprisingly, the 3-hydroxy-2-cyclohexen-1-ones exhibit a substantially better herbicidal activity than those known from the aforementioned prior art.

Preferred compounds of the formula (I), are those in which

$R^1$ represents methyl, ethyl, n-propyl, iso-propyl, n-butyl, iso-butyl, sec-butyl, tert.-butyl, cyclohexylmethyl, cyclopentylmethyl, cyclopropylmethyl, or represents allyl, 1-propenyl, 1-butenyl, 2-butenyl in each case optionally substituted by fluorine, chlorine, bromine and/or iodine, or represents propargyl, 1-butynyl and 2-butynyl,

$R^2$ represents methyl, ethyl, n-propyl, iso-propyl, n-butyl, iso-butyl, sec.-butyl, tert.-butyl,

m represents 2 or 3 and

n represents 0 or 2.

In particular, the invention relates to compounds of the formula (I) in which

$R^1$ represents methyl, ethyl, cyclopropylmethyl and allyl which may be substituted by chlorine, or represents propargyl,

$R^2$ represents methyl, ethyl, n-propyl or n-butyl,

m represents 2 or 3 and

n represents 0.

The following compounds may be, in addition to those exemplified in working examples hereinafter, mentioned:

5-[2-(3,4-difluorophenylthio)ethyl]-3-hydroxy-2-(1-methoxyiminopropyl)-2-cyclohexen-1-one,
5-[2-(3,4-difluorophenylthio)ethyl]-3-hydroxy-2-(1-ethoxyiminoethyl)-2-cyclohexen-1-one,
5-[2-(3,4-difluorophenylthio)ethyl]-3-hydroxy-2-(1-allyloxyiminoethyl)-2-cyclohexen-1-one,
5-[2-(3,4-difluorophenylthio)ethyl]-3-hydroxy-2-[1-(3-chloroallyloxyimino)ethyl]-2-cyclohexen-1-one,
5-[2-(3,4-difluorophenylthio)ethyl]-3-hydroxy-2-(1-propargyloxyiminoethyl)2-cyclohexen-1-one,
5-[2-(3,4-difluorophenylthio)ethyl]-3-hydroxy-2-(1-ethoxyiminopropyl)2-cyclohexen-1-one,
5-[2-(3,4-difluorophenylthio)ethyl]-3-hydroxy-2-[1-(3-chloroallyloxyimino)propyl]-2-cyclohexen-1-one,
5-[2-(3,4-difluorophenylthio)ethyl]-3-hydroxy-2-(1-propargyloxyiminopropyl)-2-cyclohexen-1-one,
5-[2-(3,4-difluorophenylthio)ethyl]-3-hydroxy-2-(1-allyloxyiminopropyl)-2-cyclohexen-1-one,
5-[2-(3,4-difluorophenylthio)ethyl]-3-hydroxy-2-(1-ethoxyiminobutyl)-2-cyclohexen-1-one,
5-[2-(3,4-difluorophenylthio)ethyl]-3-hydroxy-2-(1-allyloxyiminobutyl)-2-cyclohexen-1-one,
5-[2-(3,4-difluorophenylthio)ethyl]-3-hydroxy-2-[1-(3-chloroallyloxyimino)butyl]-2-cyclohexen-1-one,
5-[2-(3,4-difluorophenylsulfonyl)ethyl]-3-hydroxy-2-(1-ethoxyiminoethyl)-2-cyclohexen-1-one,
5-[2-(3,4-difluorophenylsulfonyl)ethyl]-3-hydroxy-2-(1-ethoxyiminopropyl)-2-cyclohexen-1-one,
5-[2-(3,4-difluorophenylsulfonyl)ethyl]-3-hydroxy-2-(1-ethoxyiminobutyl)-2-cyclohexen-1-one,
5-[2-(3,4-difluorophenylthio)ethyl]-3-hydroxy-2-(1-cyclopropylmethoxyiminopropyl)-2-cyclohexen-1-one.

If use is made, in the above-mentioned process a), of 5-[2-(3,4-difluorophenylthio)ethyl]-3-hydroxy-2-propionyl-2-cyclohexen-1-one and o-methylhydroxyalkylamine hydrochloride as the starting materials, for example, the course of the reaction can be represented by the following equation:

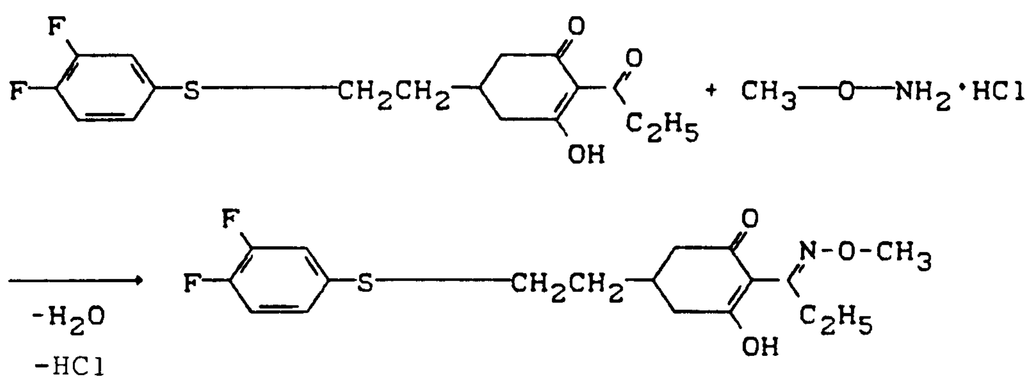


If use is made, in the above-mentioned process b), of 5-[2-(3,4-difluorophenylthio)ethyl]-3-hydroxy-2-(1-methoxyiminopropyl)-2-cyclohexen-1-one and m-chloro perbenzoic acid, as the starting materials, for example, the course of the reaction can be represented by the following equation:

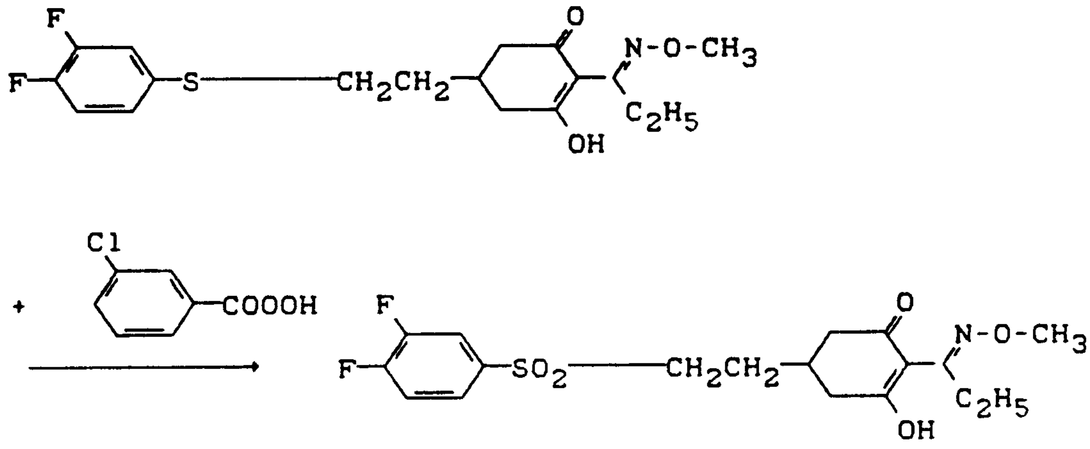

In the above-mentioned process a), the compound of the formula (II) means compounds based on the above definitions of $R^2$, m and n and preferably compounds based on the above preferred definitions.

The compounds of the formula (II) can be prepared by the process disclosed by Japanese Patent Laid-open No. 124224/1990 and the following examples may be mentioned:

5-[2-(3,4-difluorophenylthio)ethyl]-3-hydroxy-2-propionyl-2-cyclohexen-1-one, and

5-[2-(3,4-difluorophenylthio)ethyl]-3-hydroxy-2-acetyl-2-cyclohexen-1-one.

The compounds of the formula (III) mean compounds based on the above definitions of $R^1$ and, preferably those based on the above preferred definitions.

As the examples of the compounds of the formula (III) may be mentioned:

O-(3-chloro)alkylhydroxylamine,

O-methylhydroxylamine, O-ethylhydroxylamine, O-allylhydroxylamine, O-propargylhydroxylamine and their hydrochlorides.

In the above-mentioned process b), the compounds of the formula (Ia) may be included in the formula (I).

As the oxydizing agent is used in the above-mentioned process b), use is made, for example, of organic peroxides such as m-chloroperbenzoic acid, hydrogen peroxide and the like.

In conducting the process a) mentioned above, every kinds of inert solvents can be mentioned as appropriate diluents.

Examples of such diluents are water; aliphatic, cycloaliphatic and aromatic, optionally chlorinated, hydrocarbons such as hexane, cyclohexane, petroleum ether, ligroin, benzene, toluene, xylene, methylene chloride, chloroform, carbon tetrachloride, ethylene chloride, 1,2-dichloroethane, chlorobenzene, dichlorobenzene and the like; ethers such as diethyl ether, methyl ethyl ether, di-isopropyl ether, dibutyl ether, dioxane, tetrahydrofuran and the like; alcohols such as methanol, ethanol, iso-propanol, butanol, ethylene glycol, 2-methoxy ethanol and the like, and bases such as pyridine, for example.

The above-mentioned process a) is carried out preferably in the presence of acid binder. Examples of such an acid binder include, for example, hydroxides, carbonates, bicarbonates, acetates and alcolates of alkalimetals, tertiary amines such as, for example, triethylamine, tributylamine, 1,1,4,4-tetramethylethylenediamine, N,N-dimethylaniline, N,N-diethylaniline, pyridine, 4-dimethylamino pyrimidine (DMAP), 1,4-diazabicyclo[2,2,2] octane (DABCO), 1,8-diazabicyclo[5,4,0]undec-7-ene (DBU).

The above-mentioned process a), can be carried out at temperatures over a substantially wide range. In general, the reaction is carried out at a temperature of from about 0°C to about 100°C, preferably at room temperature.

Further, the reaction is desirably carried out under normal pressure, although it is possible to operate under elevated or reduced pressure.

In performing the process a) the desired compounds of the formula (I) can be obtained, for example, by reacting 1.0 to 2.0 mol amount of the compounds of the formula (III) with 1 mol of the compounds of the formula (II) in the presence of 1.0 to 2 mol amount of triethylamine dissolved in inert solvent such as methanol for example.

In conducting the process b) mentioned above, every kinds of inert solvents can be mentioned as appropriate diluents.

Examples of such diluents are aliphatic, cycloaliphatic and aromatic, optionally chlorinated, hydrocarbons such as hexane, cyclohexane, benzene, toluene, xylene, methylene chloride, chloroform, carbon

tetrachloride, ethylene chloride, 1,2-dichloroethane, chlorobenzene, dichlorobenzene and the like.

When use is made, in carrying out the above-mentioned process b), of hydrogen peroxide as oxidizing agent, any inert solvent may be used as suitable solvents.

Examples of such diluents are water that may be basic, neutral or acidic; carboxylic acids such as acetic acid, propionic acid, for example.

The above-mentioned process b) according to the present invention is carried out at temperatures over a substantially wide range. In general the reaction is carried out at a temperature of from about 50 ° C to about 100 ° C, preferably from about 80 ° C to about 100 ° C.

The reaction of the above-mentioned process b) is preferably carried out under normal pressure, although it is also possible to operate under elevated or reduced pressure.

In performing the process b), the desired compounds of the formula (I), wherein n is 1, can be obtained by reacting 1 mol amount of m-chloroperbenzoic acid with 1 mol of the compounds of the formula (Ia) in the presence of an inert solvent such as, for example, chloroform.

The active compounds according to the invention can be used as defoliants, desiccants, agents for destroying broad-leaved plants and, especially, as weedkillers. By weeds, in the broadest sense, there are to be understood all plants which grow in locations where they are undesired. Whether the substances according to the invention act as total or selective herbicides depends essentially on the amount used.

The active compounds according to the invention can be used, for example, in connection with the following plants:

Dicotyledon weeds of the genera: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver and Centaurea.

Dicotyledon cultures of the genera: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis and Cucurbita.

Monocotyledon weeds of the genera: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus and Apera.

Monocotyledon cultures of the genera: Oryza, Zea, Triticum, Hordeum, Avena, Secale, Sorghum, Panicum, Saccharum, Ananas, Asparagus and Allium.

However, the use of the active compounds according to the invention is in no way restricted to these genera, but also extends in the same manner to other plants.

The compounds are suitable, depending on the concentration, for the total combating of weeds, for example on industrial terrain and rail tracks, and on paths and squares with or without tree plantings. Equally, the compounds can be employed for combating weeds in perennial cultures, for example afforestations, decorative tree plantings, orchards, vineyards, citrus groves, nut orchards, banana plantations, coffee plantations, tea plantations, rubber plantations, oil palm plantations, cocoa plantations, soft fruit plantings and hopfields, and for the selective combating of weeds in annual cultures.

The active compounds can be converted into the customary formulations, such as solutions, emulsions, wettable powders, suspensions, powders, foams, pastes, granules, aerosols, natural and synthetic materials impregnated with active compound, very fine capsules in polymeric substances, coating compositions for use on seed, and formulations used with burning equipment, such as fumigating cartridges, fumigating cans and fumigating coils, as well as ULV cold mist and warm mist formulations.

These formulations may be produced in known manner, for example by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid solvents diluents or carriers, there are suitable in the main, aromatic hydrocarbons, such as xylene, toluene or alkyl naphthalenes, chlorinated aromatic or chlorinated aliphatic hydrocarbons, such as chlorobenzenes, chloroethylenes or methylene chloride, aliphatic hydrocarbons, such as cyclohexane or paraffins, for example mineral oil fractions, alcohols, such as butanol or glycol as well as their ethers and esters, ketones, such as acetone, methyl ethyl ketone, methyl isobutyl ketone or cyclohexanone, or strongly polar solvents, such as dimethylformamide and dimethyl-sulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

As solid carriers there may be used ground natural minerals, such as kaolins, clays, talc, chalk, quartz, attapulgite, montmorillonite or diatomaceous earth, and ground synthetic minerals, such as highly-dispersed silicic acid, alumina and silicates. As solid carriers for granules there may be used crushed and fractionated natural rocks such as calcite, marble, pumice, sepiolite and dolomite, as well as synthetic granules of inorganic and organic meals, and granules of organic material such as sawdust, coconut shells, corn cobs and tobacco stalks.

As emulsifying and/or foam-forming agents there may be used non-ionic and anionic emulsifiers, such as polyoxyethylene-fatty acid esters, polyoxyethylene-fatty alcohol ethers, for example alkylaryl polyglycol ethers, alkyl sulphonates, alkyl sulphates, aryl sulphonates as well as albumin, hydrolysis products. Dispersing agents include, for example, lignin sulphite waste liquors and methylcellulose.

Adhesives such as carboxymethylcellulose and natural and synthetic polymers in the form of powders, granules or latices, such as gum arabic, polyvinyl alcohol and polyvinyl acetate, can be used in the formulation.

It is possible to use colorants such as inorganic pigments, for example iron oxide, titanium oxide and Prussian Blue, and organic dyestuffs, such as alizarin dyestuffs, azo dyestuffs or metal phthalocyanine dyestuffs, and trace nutrients, such as salts of iron, manganese boron, copper, cobalt, molybdenum and zinc.

The formulations in general contain from 0.1 to 95 per cent by weight of active compound, preferably from 0.5 to 90 per cent by weight.

The active compounds according to the invention, as such or in the form of their formulations, can also be used, for combating weeds, as mixtures with known herbicides, finished formulations or tank mixes being possible.

Mixtures with other known active compounds, such as herbicides, fungicides, insecticides, acaricides, nematicides, bird repellents, plant nutrients and agents which improve soil structure, are also possible.

The active compounds can be used as such, in the form of their formulations or in the use forms prepared therefrom by further dilution, such as ready-to-use solutions, suspensions, emulsions, powders, pastes and granules. They are used in the customary manner, for example by watering, spraying, atomizing or scattering.

The active compounds according to the invention can be applied either before or after emergence of the plants.

They can also be incorporated into the soil before sowing. They are used, in particular, after emergence of the plants.

The amount of active compounds used can vary within a substantial range. It depends essentially on the nature of the desired effect. In general, the amounts used are between 0.01 and 4 kg of active compound per hectare of soil surface, preferably between 0.05 and 2 kg per ha.

The preparation and the use of the active compounds according to the present invention are illustrated by the following examples.

It is understood that the specification and examples are illustrative but not limitative of the present invention and that other embodiments within the spirit and scope of the invention will suggest themselves to those skilled in the art.

Preparative Example

Example 1

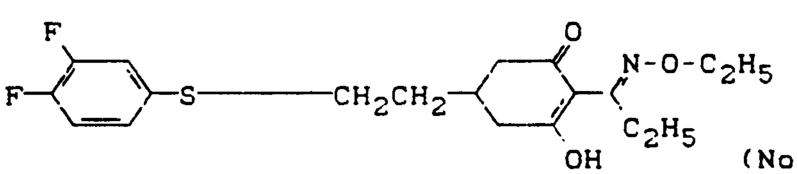

(No. 1)

A mixture of 5-[2-(3,4-difluorophenylthio)ethyl]-3-hydroxy-2-propionyl-2-cyclohexen-1-one (3.40 g), O-ethylhydroxyamine hydrochloride (1.39 g) and triethylamine (1,5 g) in methanol (50 ml) was stirred at room temperature. After twelve hours stirring the reaction mixture was poured into water and then acidified with diluted hydrochloric acid, followed by extraction with ethyl acetate. The combined organic layers were dried

over anhydrous sodium sulfate filtered and the solvent was distilled off under reduced pressure. The residue was purified through column chromatography (eluent: hexane:ethyl acetate = 85:15) to obtain 3.25 9 of 5-[2-(3,4-difluorophenylthio)ethyl]-3-hydroxy-2-(1-ethoxyiminopropyl)-2-cyclohexen-1-one. $n_D^{20}$ 1.5576.

Example 2

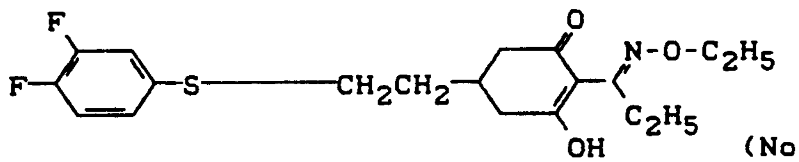

(No. 1)

5-[2-(3,4-difluorophenylthio)ethyl]-3-hydroxy-2 propionyl-2-cyclohexen-1-one (3.40 g), O-ethylhydroxyamine hydrochloride (1.39 g) and triethylamine (1.5 g) were dissolved in methanol (50 ml). The mixture was stirred for twelve hours at room temperature.

The solvent was distilled off under reduced pressure, and the resulting residue was mixed with ethyl acetate (60 ml). The organic layer was washed with diluted hydrochloric acid and water successively. Then the extract was dried over anhydrous magnesium sulfate, filtered and evaporated to give crude product.

The product was column chromatographed on silica gel (eluent; hexane:ethyl acetate = 5:1) to give 3.30 g of 5-[2-(3,4-difluorophenylthio)ethyl]-3-hydroxy-2-(1-ethoxyiminopropyl)-2-cyclohexen-1-one. $n_D^{20}$ 1.5576

The compounds which can be obtained in the same manner as the above examples are shown in Table 1.

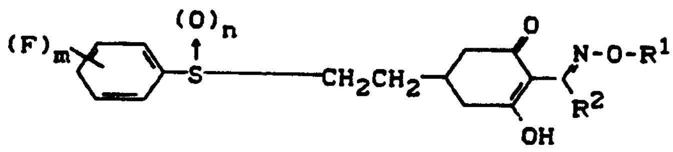 (I)

Table 1

| Comp. No. | (F) | n | $R^1$ | $R^2$ | |
|---|---|---|---|---|---|
| 2 | 3,4-$F_2$ | 0 | $C_2H_5$ | $CH_3$ | $n_D^{20}$ 1.5603 |
| 3 | 3,4-$F_2$ | 0 | $CH_2CH=CH$ | $CH_3$ | $n_D^{20}$ 1.5611 |
| 4 | 3,4-$F_2$ | 0 | $CH_2CH=CHCl$ | $CH_3$ | |
| 5 | 3,4-$F_2$ | 0 | $CH_2C\equiv CH$ | $CH_3$ | |
| 6 | 3,4-$F_2$ | 0 | $CH_3$ | $C_2H_5$ | |
| 7 | 3,4-$F_2$ | 0 | $CH_2CH=CHCl$ | $C_2H_5$ | $n_D^{20}$ 1.5584 |
| 8 | 3,4-$F_2$ | 0 | $CH_2C\equiv CH$ | $C_2H_5$ | $n_D^{20}$ 1.5431 |
| 9 | 3,4-$F_2$ | 0 | $CH_2CH=CH$ | $C_2H_5$ | $n_D^{20}$ 1.5550 |
| 10 | 3,4-$F_2$ | 0 | $C_2H_5$ | n-$C_3H_7$ | $n_D^{20}$ 1.5296 |
| 11 | 3,4-$F_2$ | 0 | $CH_2CH=CH$ | n-$C_3H_7$ | $n_D^{20}$ 1.5419 |
| 12 | 3,4-$F_2$ | 0 | $CH_2CH=CHCl$ | n-$C_3H_7$ | $n_D^{20}$ 1.5370 |
| 13 | 3,4-$F_2$ | 0 | $CH_2$ 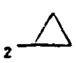 | $C_2H_5$ | |
| 14 | 3,4-$F_2$ | 2 | $C_2H_5$ | $CH_3$ | |
| 15 | 3,4-$F_2$ | 2 | $C_2H_5$ | $C_2H_5$ | |
| 16 | 3,4-$F_2$ | 2 | $C_2H_5$ | n-$C_3H_7$ | |
| 17 | 3,4-$F_2$ | 0 | $CH_2C\equiv CH$ | n-$C_3H_7$ | $n_D^{20}$ 1.5319 |
| 18 | 2,3,4-$F_3$ | 0 | $C_2H_5$ | $CH_3$ | $n_D^{20}$ 1.5743 |

Table 1 (continued)

| Comp. No. | (F) | n | $R^1$ | $R^2$ | |
|---|---|---|---|---|---|
| 19 | 2,3,4-$F_3$ | 0 | $C_2H_5$ | $C_2H_5$ | $n_D^{20}$ 1.5629 |
| 20 | 2,3,4-$F_3$ | 0 | $CH_2CH=CHCl$ | $C_2H_5$ | $n_D^{20}$ 1.5599 |
| 21 | 2,3,4-$F_3$ | 0 | $CH_2C\equiv CH$ | $C_2H_5$ | |
| 22 | 2,3,4-$F_3$ | 0 | $CH_2CH=CH_2$ | $CH_3$ | $n_D^{20}$ 1.5648 |
| 23 | 2,4-$F_2$ | 0 | $C_2H_5$ | $CH_3$ | $n_D^{20}$ 1.5562 |
| 24 | 2,4-$F_2$ | 0 | $C_2H_5$ | $C_2H_5$ | $n_D^{20}$ 1.5506 |
| 25 | 2,4-$F_2$ | 0 | $CH_2CH=CH_2$ | $C_2H_5$ | $n_D^{20}$ 1.5542 |
| 26 | 2,4-$F_2$ | 0 | $C_2H_5$ | n-$C_3H_7$ | $n_D^{20}$ 1.5184 |
| 27 | 2,4-$F_2$ | 0 | $CH_2CH=CH_2$ | $CH_3$ | $n_D^{20}$ 1.5581 |

Biotest Example:

Comparative Compound:

C-1

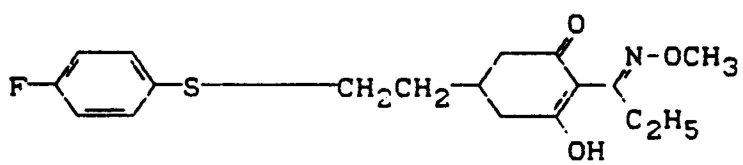

(Disclosed by Japanese Patent Laid-open No. 14685/1988)

Test Example 1

Pre-emergence soil treatment on upland weeds

Formulation of Active Compounds

Carrier: 5 parts by weight of acetone
Emulsifier: 1 part by weight of benzyloxy polyglycol ether

To produce a suitable preparation of active compound, 1 part by weight of each of the active compounds according to the present invention was mixed with the stated amount of carrier and the stated amount of emulsifier, and the resulting emulsifiable concentrate was then diluted with water to the desired concentrations.

Test Procedures

In a greenhouse, a number of test pots each having an area of 1000 $cm^2$ were charged with soil taken out from a cultivated field.

Onto the soil surfaces in the respective test pots were sown the seeds of barnyards grass (Echinochloa crusgalli), fingergrass (Digitaria adsendeus), green foxtail (Setaria viridis), and dent foxtail (Alopecurus aequalis), together with the seeds of soy beans and cotton, respectively, followed by soil covering thereon. Thereafter, predetermined dosages of the active compound formulations mentioned above were uniformly sprayed onto the soil surfaces of the respective test pots.

Four weeks after the application of the active compound formulations, the degrees of herbicidal effect on the weeds and the degrees of the phytotoxicity on the crop were determined based on the following assessment:

A herbicidal effect on 100%

was rated as "completely killed" and the determined degrees of herbicidal effects were rated under the corresponding percentage.

A clearly superior effectiveness combined with good selectivity towards crop plants compared with the known compound C-1 is shown in this test, for example, by the Preparation Examples 1, 2, 3, 7, 9, 10 and 19.

Test Example 2

Post-emergence foliage treatment on upland weeds

In a greenhouse, a number of test pots each having an area of 2000 $cm^2$ were charged with soil taken out from a cultivated field. Seeds of soy bean were sown onto the soil surfaces in the respective test pots. Each of the thus sown soil surfaces was covered with a soil layer to a height of 1 cm. The soil of the respective soil layers in the respective test pots had beforehand been mixed with the seeds of barnyard grass, finger grass, goose grass, green foxtail, causeway grass (Poa annua) and Bermuda grass (Cynodon dactylon), respectively.

Ten days after the seed-sowing and soil-covering when the weeds entered the second leaf stage on average while the soy beans entered the early leafing stage of true leaves, predetermined dosages of the active compound formulations prepared as in Example 1 mentioned above were uniformly sprayed onto the foliage portions of the test weeds in the respective test pots.

Three weeks after the spraying of the active compound formulations, the degrees of the herbicidal effect on the weeds and the degrees of the phytotoxicity on the crop were determined.

A clearly superior effectiveness (combined with good selectivity towards crop plants) compared with the known compound C-1 is shown in this test, for example, by the compounds of Preparation Examples 1, 2, 3, 7 and 10.

Test Example 3:

Test for determining herbicidal effect on lowland weeds

Onto a plurality of pots (25 x 20 x 9 cm) each having

$$\frac{1}{2000}$$

are filled with paddy field soil, while paddy rice seedlings (species: NIHONBARE) at 2.5 leave stage (plant height of about 15 cm) were transplanted with three seedlings per unit at two separate sections in each of the pots.

Further, seeds of Barnyard grass were sown into the respective pots and they were kept in wet conditions. Two days after, the pots were filled with water to the height of about 2 to 3 cm above the soil surfaces and five days after the paddy rice transplantation, the predetermined dosages of the active compound mixtures according to the present invention, formulated according to the formulation similar to Example 1 were applied to the respective pots according to water-surface treatment with the use of a pipette.

After the treatment, the water layer in each of the pots was kept at a height of about 3 cm and four weeks after the treatment, the herbicidal effects were evaluated.

A clearly superior effectiveness compared with the known compound C-1 is shown in this test, for example, by the compounds of Preparation Examples 22 and 23.

## Claims

1. 3-hydroxy-2-cyclohexen-1-ones of the formula (I)

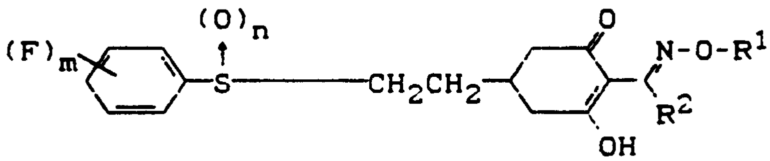

(I)

wherein

$R^1$ represents $C_{1-4}$-alkyl group, cycloalkylalkyl with up to 6 carbon atoms in the cycloalkyl moeity and up to 4 carbon atoms in the alkyl moeity, $C_{3-4}$ alkenyl which may be substituted by halogen or $C_{3-4}$ alkynyl group,
$R^2$ represents $C_{1-4}$-alkyl group,
m represents 2 or 3, and
n represents 0 or 2.

2. The compounds of the formula (I) according to claim 1 wherein
$R^1$ represents methyl, ethyl, n-propyl, iso-propyl, n-butyl, iso-butyl, sec-butyl, tert.-butyl, cyclohexylmethyl, cyclopentylmethyl, cyclopropylmethyl, or represents allyl, 1-propenyl, 1-butenyl, 2-butenyl in each case optionally substituted by fluorine, chlorine, bromine and/or iodine, or represents propargyl, 1-butynyl and 2-butynyl,
$R^2$ represents methyl, ethyl, n-propyl, iso-propyl, n-butyl, iso-butyl, sec-butyl, tert-butyl
m represents 2 or 3 and
n represents 0 or 2.

3. The compounds of the formula (I) according to claim 1 wherein
$R^1$ represents methyl, ethyl, cyclopropylmethyl and allyl which may be substituted by chlorine, or represents propargyl
$R^2$ represents methyl, ethyl, n-propyl or n-butyl,
m represents 2 or 3 and
n represents 0.

4. Process for the preparation of the compounds of the formula (I)

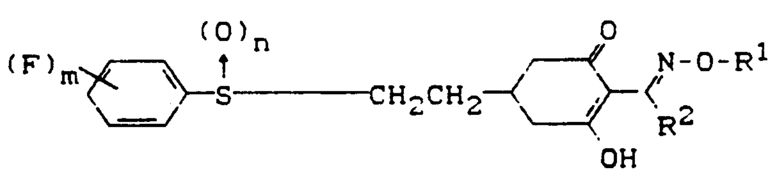

(I)

wherein

$R^1$ represents $C_{1-4}$-alkyl group, cycloalkylalkyl with up to 6 carbon atoms in the cycloalkyl moeity and up to 4 carbon atoms in the alkyl moeity, $C_{3-4}$ alkenyl which may be substituted by halogen or $C_{3-4}$ alkynyl group,
$R^2$ represents $C_{1-4}$-alkyl group,
m represents 2 or 3, and
n represents 0 or 2,

characterized in that

a) compounds of the formula (II)

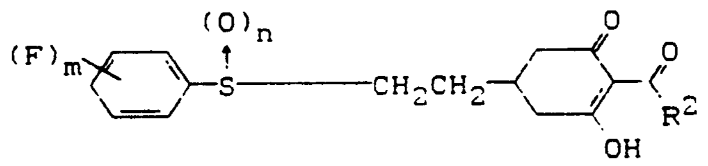

(II)

wherein

$R^2$, m and n have the same meanings as mentioned above,

are reacted with compounds of the formula (III)

$$R^1—O—NH_2 \quad (III)$$

wherein

$R^1$ has the same meaning as mentioned above, in the presence of inert solvents,

or
b) where n is 2:

compounds of the formula (Ia)

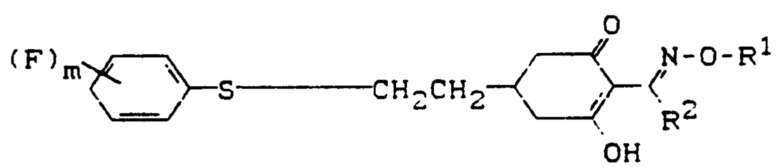

(Ia)

wherein $R^1$, $R^2$ and m have the same meanings as mentioned above,

are oxidized in the presence of inert solvents.

**5.** Herbicidal compositions, characterized in that they contain at least one 3-hydroxy-2-cyclohexen-1-one of the formula (I) according to claim 1.

**6.** Process for combating weeds, characterized in that 3-hydroxy-2-cyclohexen-1-ones of the formula (I), according to claim 1, are allowed to act on weeds and/or their habitat.

**7.** Use of 3-hydroxy-2-cyclohexen-1-ones of the formula (I), according to claim 1, for combating weeds.

**8.** Process for the preparation of herbicidal compositions, characterized in that 3-hydroxy-2-cyclohexen-1-ones of the formula (I), according to claim 1, are mixed with extenders and/or surface active agents.

European Patent Office

# EUROPEAN SEARCH REPORT

Application Number

EP 92 11 1990

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,X | GB-A-1 589 003 (NIPPON SODA CO. LTD.)<br>* page 18; claim 1 *<br>* page 1, line 5 - line 6 * | 1-3,7 | C07C323/47<br>C07C317/28<br>A01N41/10<br>A01N35/10 |
| D,A | EP-A-0 254 514 (SUMITOMO CHEMICAL COMPANY, LIMITED)<br>* the whole document * | 1-3,7 | |
| A | EP-A-0 262 265 (CHEVRON RESEARCH COMPANY)<br>* page 13; claim 1 *<br>* page 7, line 56 *<br>* page 3, line 1 - line 2 * | 1-3,7 | |
| A | CHEMICAL ABSTRACTS, vol. 111, no. 17, 23 October 1989, Columbus, Ohio, US; abstract no. 153356f,<br>K. ARAI ET AL. 'Preparation of 5-[2-(phenylthio)ethyl]cyclohexane-1,3-diones as herbicides.'<br>page 675 ;column 2 ;<br>* abstract *<br>& JP-A-0 129 355 (SUMITOMO CHEMICAL COMPANY, LIMITED)<br>31 January 1989 | 1,7 | TECHNICAL FIELDS SEARCHED (Int. Cl.5)<br>C07C |

The present search report has been drawn up for all claims

| Place of search | Date of completion of the search | Examiner |
|---|---|---|
| THE HAGUE | 23 SEPTEMBER 1992 | FINK D.G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)